# EUROPEAN PATENT SPECIFICATION

(11) **EP 1 395 206 B1**
(45) Date of publication and mention of the grant of the patent: **18.06.2008**
(21) Application number: 01947786.8
(22) Date of filing: 15.06.2001
(51) Int. Cl.: A61F 2/34

(54) **ACETABULAR CUP FOR HIP JOINT PROSTHESIS**
GELENKPFANNE FÜR HÜFTPROTHESE
COTYLE POUR PROTHESE D'ARTICULATION DE HANCHE

(43) Date of publication of application: 10.03.2004
(73) Proprietor: Permedica S.p.A., 23807 Merate (Lecco) (IT)
(72) Inventor: PEREGO, Marco, I-23807 Merate (IT)
(74) Representative: Siniscalco, Fabio
(86) International application number: PCT/IT2001/000320
(87) International publication number: WO 2002/102285

(56) References cited:
- EP-A- 0 190 093
- EP-A- 1 066 806
- EP-A- 1 086 666
- WO-A-99/60955
- DE-C- 19 654 409
- US-A- 5 133 764
- US-A- 5 919 236

## Description

The subject of the present invention is an acetabular cup for a hip joint prosthesis which is suitable for receiving both inserts made of ceramic material and inserts made of synthetic material.

Hip joint prostheses comprising, amongst other things, an acetabular cup and a cap-shaped insert, are known. The acetabular cup is intended to be fitted in a suitable seat formed in the region of the acetabulum by surgical intervention. The cup defines an internal cavity in which the cap-shaped insert, which performs the role of the acetabulum, housing the substantially spherical head of a femoral prosthesis, is fitted.

The acetabular cup is generally made of a biocompatible and osteo-integratable material such as, for example, titanium and some of its alloys. The cap-shaped insert is usually made of a material with good wear-resistance and a low coefficient of friction in the coupling with the above-mentioned head of the femoral prosthesis. Materials commonly used for the production of these inserts are polyethylene with a high molecular density, as well as some ceramic materials.
EP 1 086 666 ***discloses a socket for a hip joint prosthesis according to the preamble of claim 1*.**

However, there are some problems and disadvantages in the coupling of the insert with the acetabular cup, due both to the specific properties and diversity of the materials used and to the mechanical stresses to which they are subjected.

With polyethylene inserts, play due to wear and/or deformation of the polyethylene may arise between the insert and the acetabular cup over time. Extreme consequences of this play may be rotation of the insert in the cavity of the acetabular cup as well as expulsion of the insert because of the resilience properties of the material.

As well as adversely affecting the operation of the prosthesis, the play which arises over time also leads to a change in the expected effect of the forces between the bony structure and the individual components of the hip joint prosthesis, leading to secondary damage of various kinds.

Whereas polyethylene inserts present the problem of wear and of play in the coupling with the acetabular cup, ceramic inserts lead to difficulties connected with the high degree of fragility of these materials. In fact, inserts made of ceramic material require a very precise geometrical coupling with the acetabular cup to prevent cracking and brittle breakage as a result of non-uniform lads and possible concentrations of stresses.

The above-mentioned reasons for damage to the joint inserts and for undesired play arising in the coupling between the acetabular cup and the joint insert constitute a very serious problem, since a further surgical operation on the patient is inevitable in order to re-establish the correct operative condition of the prosthesis.

A further drawback of the prior art is connected with the fact that joint inserts of different materials require different shaping and machining of the cavity inside the acetabular cup. It is-therefore necessary to have different acetabular cups available, according to the material of which the corresponding insert is made. As well as a danger of confusion, this also leads to considerable constructional complexity and high production costs.

The aim of the present invention is therefore to solve the problems set out above by providing an acetabular cup for a hip joint prosthesis which has characteristics such that it can house both joint inserts made of ceramic material and joint inserts made of synthetic material, particularly polyethylene, but which satisfies the specific requirements of the two materials in order to ensure a secure and reliable coupling.

The aim set out above is achieved by means of a socket for a hip joint prosthesis as defined in claim 1.

Preferred embodiments are disclosed in dependent claims 2-24.

For a better understanding of the invention, a nonlimiting embodiment thereof is described below and illustrated in the appended drawings, in which:
Figure 1 is a proximal front view of the acetabular cup according to the invention,
Figure 2 shows the acetabular cup of Figure 1 in a cross-section taken on the line II-II,
Figure 3 is a proximal front view of the joint insert made of synthetic material according to the invention,
Figure 4 shows the joint insert of Figure 3 in a cross-section taken on the line IV-IV,
Figure 5 is a proximal front view of a joint insert made of ceramic material,
Figure 6 shows the joint insert of Figure 5 in a cross-section taken on the line VI-VI,
Figure 7 shows a section taken on the line VII-VII of Figure 2,
Figure 8 shows the detail VIII of Figure 2 in an enlarged cross-section,
Figure 9 shows, in cross-section, the coupling between an acetabular cup according to the invention and an insert made of synthetic material according to the invention,
Figure 10 shows, in cross-section, the coupling between an acetabular cup according to the invention and a conventional insert made of ceramic material, and
Figure 11 shows schematically a hip joint prosthesis with an acetabular cup according to the invention.

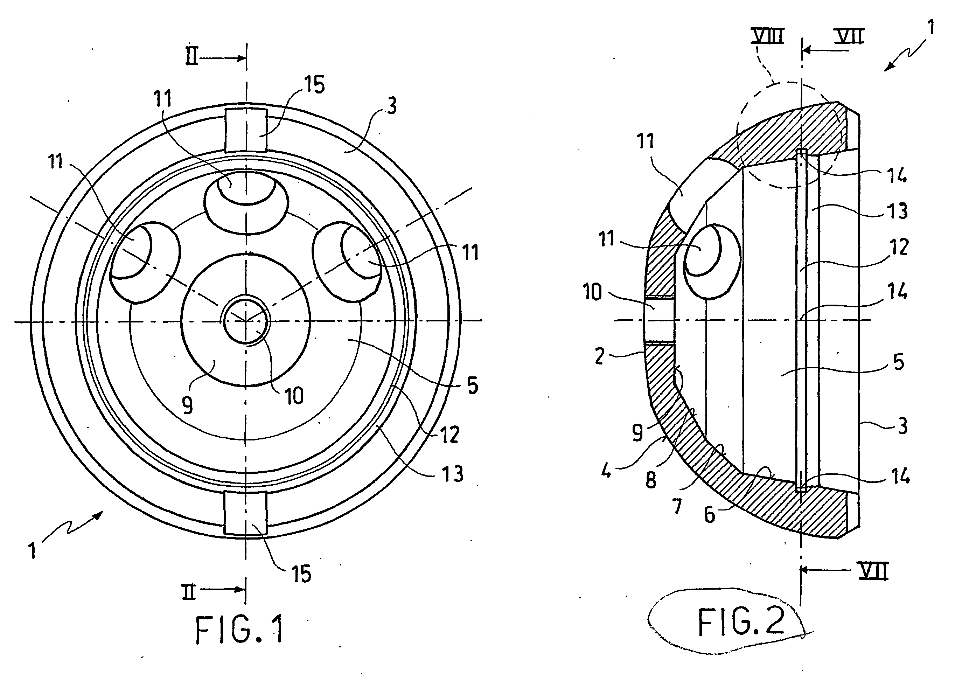

The acetabular cup shown in Figures 1 and 2 comprises a socket body 1 made of biocompatible and osteo-integratable material.

The socket 1 has a substantially spherical, cap-like shape having a pole 2 and an equatorial circumference 3 which constitute the distal end and the proximal end of the cap, respectively. The socket 1 comprises a distal surface 4 and a proximal cavity 5.

The distal surface 4 is substantially spherical, except in the vicinity of the pole 2, where it is flattened. The surface 4 is also machined so as to have a high degree of roughness.

The proximal cavity 5 is composed of 3 frustoconical portions 6, 7, 8 which are tapered successively towards the pole 2, as well as a substantially flat polar base 9.

According to one embodiment, the socket 1 has a radial through-hole 10 in the region of the pole 2, the hole 10 being internally threaded and flared in the region of the polar base 9.

According to a further embodiment, three further through-holes 11 are formed substantially radially in the frustoconical portions 7 and 8. The holes 11 have a deep, rounded flare which extends almost throughout their depth.

The frustoconical portion 6 has a knurled surface in which the raised portions of the knurling extend circumferentially, parallel to the equatorial circumference 3 of the socket 1. Moreover, the frustoconical portion 6 is interrupted, approximately centrally relative to its height, by a channel 12 which is also substantially circumferential and parallel to the equatorial circumference 3.

The channel 12 has a substantially rectangular shape in cross-section, and a depth of the same order of magnitude as its width.

According to one embodiment, the width of the channel is 1.1 mm and its maximum depth is at least 1.2 mm.

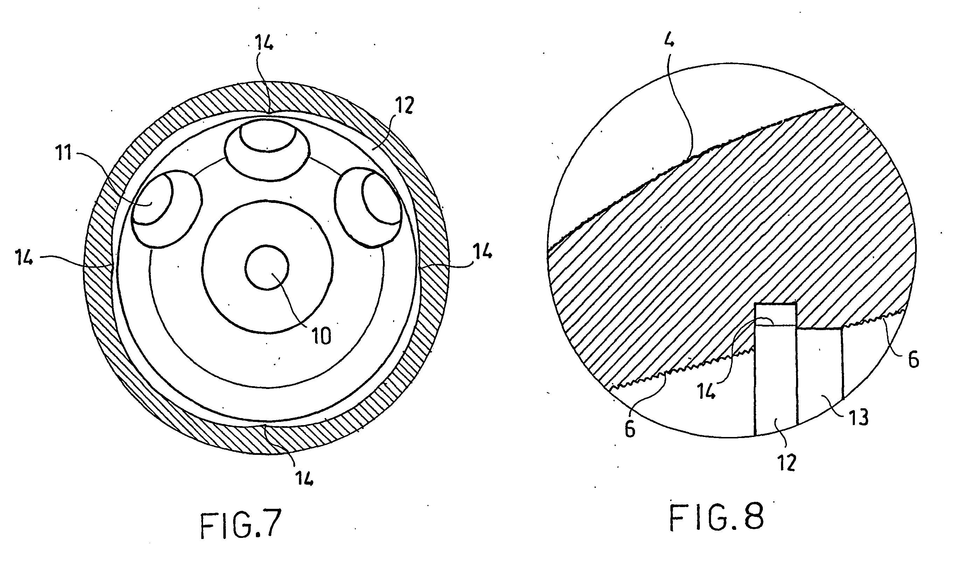

The depth of the channel 12 is not uniform but has, for example, a substantially epicycloidal shape with four cusps 14 distributed uniformly around its circumference, as can be seen from Figure 7.

Beside the channel 12 in a proximal direction, is a substantially cylindrical recess 13, the diameter of which corresponds to the diameter of the immediately adjacent frustoconical portion 6.

The channel 12 and the cylindrical recess 13 together extend around the entire circumference of the frustoconical portion 6 but cover only a small fraction of the overall surface area of the frustoconical portion 6.

Two shallow channels 15 are formed on two opposed sides of the proximal rim of the socket 1 and extend throughout the thickness of the rim of the socket 1.

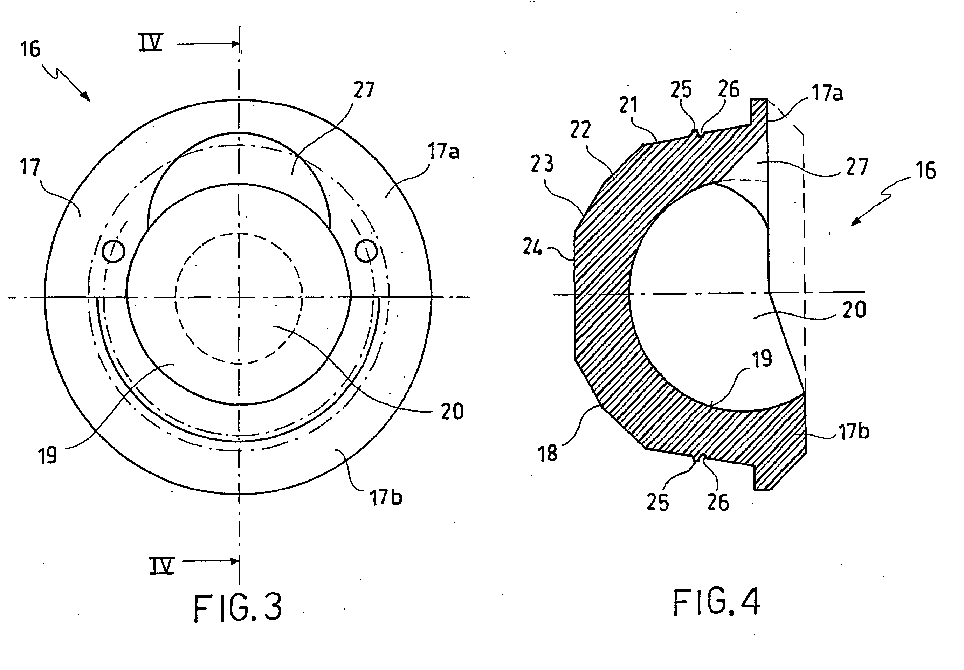

Figures 3 and 4 show a polyethylene joint insert 16 according to the invention.

The insert 16 is substantially cap-shaped, having a proximal flange 17 as well as an outer surface 18 and an inner surface 19. The inner surface 19, which is formed so as to be smooth, defines a substantially spherical cavity, hereinafter referred to as the joint seat 20.

The outer surface 18 is composed of three frustoconical portions 21, 22, 23 which are tapered successively towards a flattened polar region 24.

The most proximal frustoconical portion 21 has a finely knurled surface in which the raised portions of the knurling extend circumferentially and parallel to the proximal flange 17.

Moreover, the frustoconical portion 21 is interrupted approximately centrally relative to its height by an annular projection 25 which is also substantially circumferential and parallel to the proximal flange 17.

The annular projection 25 has a substantially trapezoidal cross-section in which the distal side has a greater inclination than the proximal side. The entire annular projection 25 is consequently inclined in the proximal direction.

Beside the annular projection 25 in the proximal direction, is a groove 26 which extends around the entire circumference of the frustoconical portion 21. The groove 26 has a rounded cross-section. The annular projection 25 and the groove 26 together extend around the entire circumference of the frustoconical portion 21 but cover only a small fraction of the overall surface area of the frustoconical portion 21.

According to one embodiment, the proximal flange 17 is divided substantially centrally into two portions 17a and 17b. The portion 17a of the flange 17 has a uniform thickness and a width which is tapered owing to a local flare 27 in the rim, between the flange 17 and the joint cavity 20. The portion 17b, on the other hand, has a region which is reinforced and enlarged in the proximal direction, increasing the internal surface 19 and enabling the joint seat 20 to resist dislocation.

According to one embodiment, the proximal flange 17 has two holes which can house two pins of a locating instrument.

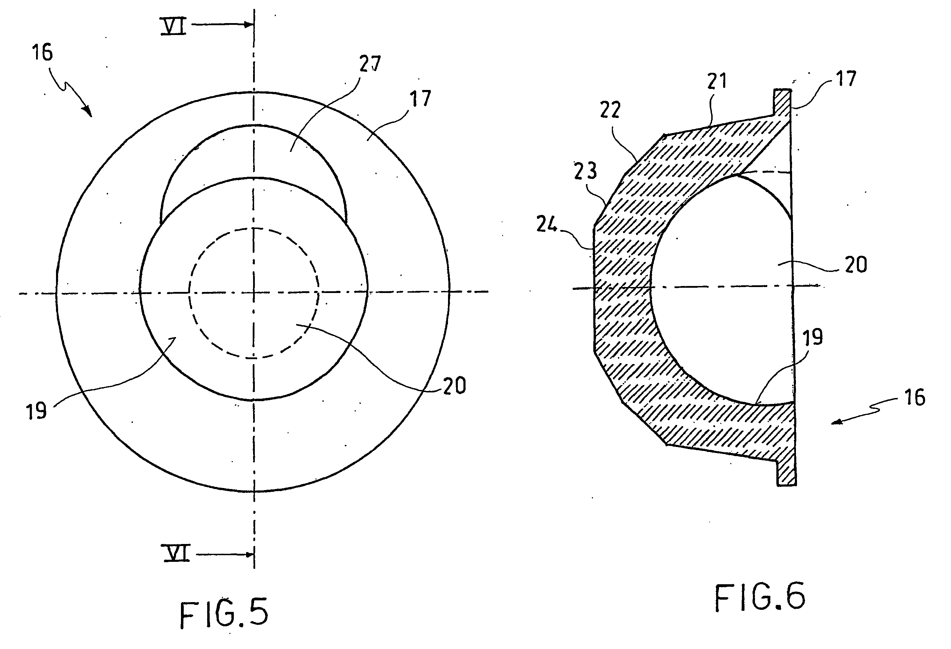

In contrast with the joint insert made of synthetic material (Figure 4), the frustoconical portion 21 of the outer surface 18 of a joint insert 16 made of ceramic material remains uninterrupted and does not have any projections or grooves (Figures 5 and 6).

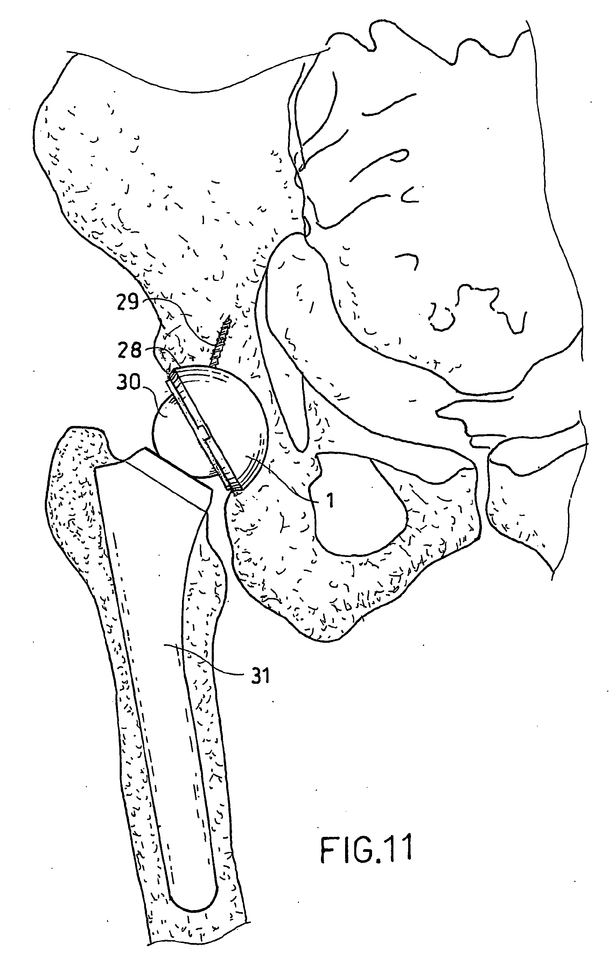

The operation of the acetabular cup 1 for hip prostheses is described below with reference to Figures 8, 9, 10 and 11.

After a natural seat 28 in the pelvic bone has been milled to a hemispherical shape, the socket 1 is inserted in the seat 28, forming a press-fit coupling (an interference fit). The socket 1 is positioned precisely by means of a suitable instrument in the form of a handle with a grip which is engaged in the threaded hole 10 in the polar base 9 of the socket. A substantially peripheral distribution of the forces acting on the socket 1 is achieved by virtue of the polar flattening of the socket 1. The tapered holes 11 can house screws for further anchorage of the socket 1 in the acetabulum. The roughness of the distal surface 4 of the socket, on the other hand, favours secondary, biological anchorage.

After the socket 1 has been positioned correctly and fixed in the natural seat 28, the joint insert 16 is inserted in the proximal cavity of the socket 1. This operation can be facilitated by means of a locating instrument with pins which engage suitable holes in the proximal flange 17.

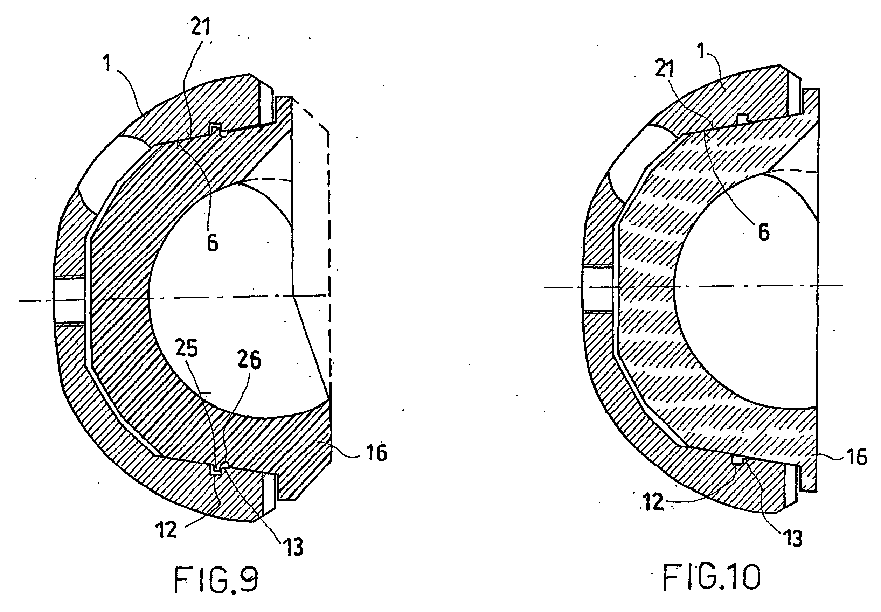

Figure 9 shows a polyethylene joint insert positioned in the acetabular cup according to the invention. During the insertion of the joint insert 16 in the socket 1, the annular projection 25 which is present on the outer surface 18 of the insert 16 bends in the proximal direction. By virtue of the groove 26, the projection 25 is not subject to a risk of cutting or plastic deformation but can deform freely and substantially resiliently, temporarily filling the space in the groove 26. At the moment at which the position of the projection 25 corresponds to the position of the channel 12, the projection 25 tends to regain its natural shape and snaps into the channel 12, preventing the joint insert 16 from coming out of the socket 1.

By virtue of the incompatibility between the advantageously epicycloidal shape of the base of the channel 12 and the uniform height of the projection 25, the cusps 14 which are present in the base of the channel 12 are impressed into the material of the projection 25, giving rise to an engagement which prevents rotation of the joint insert 16 inside the socket 1.

The polar flattening of the joint insert 16 creates a space between the respective polar regions of the insert 16 and of the proximal cavity of the socket 1. This space favours the peripheral distribution of the forces and creates a defined coupling between the socket 1 and the joint insert 16, that is, between the frustoconical portion 6 of the proximal cavity 5 and the frustoconical portion 21 of the outer surface 18.

Figure 10 shows a ceramic joint insert 16 according to the prior art positioned in the acetabular cup according to the invention.

The joint insert 16 made of ceramic material is generally inserted by pressure in the distal direction, during which the outer surface 18 immediately engages with the fine knurling of the frustoconical portion 6 of the socket 1. Further movement of the ceramic insert relative to the socket 1 is prevented by virtue of the friction between the ceramic surface and the knurling.

As with the synthetic inserts, the polar flattening of the insert 16 ensures a well-defined coupling between the frustoconical portion 6 of the socket 1 according to the invention and the frustoconical portion 21 of the ceramic joint insert 16.

The provision of the recess 13 proximally adjacent the channel 12 means that, during the insertion of the joint insert 16 in the socket 1, the annular projection 25 is not cut or deformed irreversibly before snapping into the channel 12.

The provision of the recess 13 adjacent the channel 12 also removes any concentrations of forces in the ceramic material and consequently eliminates the risk of cracking and breakage of the joint insert 16.

The approximately central arrangement of the channel 12 as well as of the recess 13 relative to the height of the frustoconical portion 6 also favours an unusually homogeneous distribution of the coupling forces. This effect is further enhanced by virtue of the limitation of the overall width of the channel 12 and of the recess 13 to a value of less than one quarter of the height of the frustoconical portion 6.

The joint insert 16 itself is intended to house a spherical head 30 of a femoral-prosthesis 31 in its joint seat 20.

The grooves 15 in the proximal rim 3 of the socket 1 afford access for tools for extracting the joint insert 16 from the proximal cavity 5 of the socket 1 for replacement or repositioning.

The acetabular cup 1 and the joint insert 16 for a hip joint prosthesis have many advantages.

The combination of a socket according to Claim 1 and a joint insert according to Claim 18 achieves the unusual advantage of preventing all of the problems mentioned with reference to the prior art.

By virtue of the structure of the socket 1 according to the invention, it is possible to use both joint inserts 16 made of ceramic material and joint inserts made of synthetic material, particularly polyethylene, with a single type of socket.

The provision of a recess 13 adjacent the channel 12 prevents the formation of concentrations of forces in ceramic joint inserts and inhibits cracking or brittle fracture of the insert during insertion and during use.

The polar flattening of the joint insert 16 favours a peripheral distribution of the coupling forces between the socket 1 and the insert 16 and ensures a well-defined bearing surface, limited to the respective frustoconical portions 6 and 21.

The fine knurling of the frustoconical portion 6 of the socket 1 ensures friction between the frustoconical portion 6 and the frustoconical portion 21 of the insert 16 made of ceramic material.

The formation of the base of the channel 12 with an epicycloidal shape prevents, to an unusual extent, any rotation of a synthetic, particularly polyethylene, joint insert inside the proximal cavity 5 of the socket 1. The absence of rotational movement also leads to very low values of wear in the connection between the socket 1 and the joint insert 16.

The engagement of the projection 25 in the channel 12 prevents expulsion of the polyethylene joint insert 16 due to resilient preloading of various origins.

The formation of a groove 26 beside the projection 25 in the polyethylene joint insert 16 allows the projection 25 to bend freely during the insertion of the joint insert in the socket and thus avoids the risk of cutting and irreversible deformation of the projection 25.

The polar flattening of the socket body 1 favours the peripheral distribution of the coupling forces between the socket 1 and the acetabulum. The polar flattening also allows the socket 1 to be forced into the natural seat 28 in which it is held in position by virtue of the annular force exerted by the bony structure.

The roughness of the distal surface 4 of the socket 1 permits optimal secondary anchorage, offering a large surface area for attachment to the bony tissue which is regenerated after the surgical operation.

Naturally, variations and/or additions may be provided for the embodiments described and illustrated above, without departing from the scope of the invention.

For example, the number and arrangement of the channels 12 in the frustoconical portion 6 as well as of the projections 25 in the frustoconical portion 21 may vary according to requirements for the fixing of the joint insert 16. The channels 12 and the projections 25 preferably extend circumferentially and, with further advantage, the channels 12 and the projections 25 form closed rings.

According to one embodiment of the invention, the channel 12 and the recess 13 are arranged approximately centrally relative to the height of the frustoconical portion 6 and their overall width is advantageously limited to a value of less than one quarter of the height of the frustoconical portion 6. With further advantage, the overall width of the channel 12 and the recess 13 is limited to a value of less than one fifth of the height of the frustoconical portion 6, and with yet further advantage, the overall width of the channel 12 and of the recess 13 is between one fifth and one sixth of the height of the frustoconical portion 6. Clearly, in order to be coupled, the respective projection 25 and the groove 26 must be arranged in a similar manner on the frustoconical portion 21 of the synthetic joint insert 16.

The number of raised portions 14 in the base of the channel 12 is variable. In one embodiment of the invention, two raised portions are provided in substantially opposed positions. The base of the channel 12 advantageously has an epicycloidal shape with four cusps 14 distributed uniformly around the circumference. The preferred embodiment of the invention provides for three cusps spaced apart uniformly by angles of 120°.

The number of holes 11 for the fixing of the socket 1 by means of screws 29 may vary according to the specific requirements of the surgical operation. The holes 11 may even be omitted. In this particular embodiment, the connection between the natural seat 28 and the socket 1 is purely of the press-fit type.

The socket 1 may be made of- any biocompatible and osteo-integratable material which is not subject to corrosion and which has a high degree of shape stability and mechanical strength. The socket according to the invention is advantageously made of titanium or titanium alloy.

The roughness of the distal surface 4 of the socket 1 is advantageously formed by the technique of plasma-spraying under vacuum or alternatively by corundum-blasting.

The enlargement of the portion 17b of the proximal flange 17 may also be omitted if a joint seat which resists dislocation is not required.

Naturally, in order to satisfy contingent and specific requirements, a person skilled in the art may apply to the acetabular cup for a hip joint prosthesis according to the present invention further modifications and variations all of which, however, are included within the scope of the invention as defined by the appended claims.

## Claims

1. A socket (1) for a hip joint prosthesis, the socket (1) being made of biocompatible and osteo-integratable material, and having a substantially spherical, cap-like shape with a distal surface (4) and a proximal cavity (5) suitable for housing both joint inserts (16) made of synthetic material and joint inserts (16) made of ceramic material, **characterized in that** the cavity (5) comprises frustoconical portions (6, 7, 8), wherein the most proximal frustoconical portion (6) has at least one substantially circumferential channel (12) which can be coupled with at least one respective projection (25) extending from an outer surface (18) of a joint insert (16) made of synthetic material, **characterized in that** the most proximal frustoconical portion (6) has a substantially cylindrical recess (13) adjacent the channel (12).

2. A socket (1) according to Claim 1 in which the recess (13) is disposed on the proximal side of the channel (12).

3. A socket (1) according to Claim 2 in which the diameter of the recess (13) corresponds to the diameter of the immediately adjacent frustoconical portion (6).

4. A socket (1) according to Claim 3 in which the channel (12) and the recess (13) are disposed approximately centrally relative to the height of the frustoconical portion (6).

5. A socket (1) according to Claim 4 in which the overall width of the channel (12) and of the recess (13) is less than one fifth of the height of the frustoconical portion (6).

6. A socket (1) according to Claim 5 in which the overall width of the channel (12) and of the recess (13) is between one fifth and one sixth of the height of the frustoconical portion (6).

7. A socket (1) according to any one of the preceding claims in which the base of the channel (12) has at least one raised portion (14) constituting means for resisting rotation.

8. A socket (1) according to Claim 7 in which the base of the channel (12) has, around its circumference, a substantially epicycloidal shape, forming at least one cusp (14).

9. A socket (1) according to Claim 8 in which the base of the channel (12) has four cusps (14) distributed substantially uniformly around its circumference.

10. A socket (1) according to Claim 8 in which the base of the channel (12) has two cusps (14) in substantially opposed positions.

11. A socket (1) according to Claim 7 in which the base of the channel (12) has three cusps spaced apart by angles of 120°.

12. A socket (1) according to any one of the preceding claims in which the polar region (2) of the distal surface (4) is flattened.

13. A socket (1) according to any one of the preceding claims in which the distal surface (4) has a high degree of roughness.

14. A socket (1) according to Claim 13 in which the roughness is produced by the technique of plasma-spraying under vacuum.

15. A socket (1) according to Claim 13 in which the roughness is produced by corundum-blasting.

16. A socket (1) according to Claim 1, having three suitably flared radial holes (11) for housing screws (29) for the anchorage of the socket (1) in the bony tissue.

17. A socket (1) according to any one of the preceding claims, made of titanium or titanium alloy.

18. A hip joint prosthesis comprising a socket (1) according to claim 1 and a joint insert (16) made of synthetic material having a substantially cap-like shape and comprising a joint seat (20) for housing a spherical head (30) of a femoral prosthesis (31), and an outer surface (18) composed of frustoconical portions (21, 22, 23) which are tapered towards a polar region (24), wherein the most proximal frustoconical portion (21) has a substantially annular projection (25) which can engage a channel (12) provided in a socket (1) for the coupling of the joint insert (16) with the socket (1), **characterized in that**, a groove (26) is formed beside the annular projection (25) in a proximal direction, and extends around the entire circumference of the frustoconical portion (21).

19. A hip joint prosthesis according to Claim 18 in which in said joint insert (16) there are three frustoconical portions (21, 22, 23).

20. A hip joint prosthesis according to Claim 19 in which in said joint insert (16) the substantially annular projection (25) is disposed approximately centrally relative to the height of the most proximal frustoconical portion (21).

21. A hip joint prosthesis according to any one of Claims 18 to 20 in which in said joint insert (16) the projection (25) is inclined in the proximal direction.

22. A hip joint prosthesis according to Claim 18, in which said joint insert (16) comprises a proximal flange (16).

23. A hip joint prosthesis according to Claim 22 in which in said joint insert (16) the proximal flange (16) has a region which is reinforced and enlarged in the proximal direction, for enabling the joint seat (20) to resist dislocation.

24. A hip joint prosthesis according to any one of Claims 18 to 23, in which said joint insert (16) is made of polyethylene.

## Patentansprüche

1. Gelenkpfanne (1) für eine Hüftprothese, wobei die Gelenkpfanne (1) aus biokompatiblen und osteointegrierbarem Material hergestellt ist und eine im Wesentlichen kugelförmige, kappenartige Form mit einer distalen Fläche (4) und einem proximalen Hohlraum (5) aufweist, der zur Aufnahme von Gelenkeinsätzen (16) sowohl aus synthetischem Material als auch von Gelenkeinsätzen (16) aus Keramikmaterial geeignet ist, **dadurch gekennzeichnet, dass** der Hohlraum (5) kegelstumpfförmige Bereiche (6, 7, 8) enthält, wobei der am proximalsten angeordnete kegelstumpfförmige Bereich (6) zumindest einen im Wesentlichen umlaufenden Kanal (12) hat, der mit zumindest einem zugehörigen Vorsprung (25) gekoppelt sein kann, der von einer Außenseite (18) eines aus einem synthetischen Material hergestellten Gelenkeinsatzes (16) verläuft und **dadurch gekennzeichnet ist, dass** der am proximalsten angeordnete kegelstumpfförmige Bereich (6) eine im Wesentlichen zylindrische Aussparung (13) neben dem Kanal (12) aufweist.

2. Gelenkpfanne (1) gemäß Anspruch 1, wobei die Aussparung (13) auf der proximalen Seite des Kanals (12) angeordnet ist.

3. Gelenkpfanne (1) gemäß Anspruch 2, wobei der Durchmesser der Aussparung (13) dem Durchmesser des direkt angrenzenden kegelstumpfförmigen Bereichs (6) entspricht.

4. Gelenkpfanne (1) gemäß Anspruch 3, wobei der Kanal (12) und die Aussparung (13) in etwa mittig relativ zur Höhe des kegelstumpfförmigen Bereichs (6) angeordnet sind.

5. Gelenkpfanne (1) gemäß Anspruch 4, in der die Gesamtbreite des Kanals (12) und der Aussparung (13) weniger als ein Fünftel der Höhe des kegelstumpfförmigen Bereichs (6) beträgt.

6. Gelenkpfanne (1) gemäß Anspruch 5, in der die Gesamtbreite des Kanals (12) und der Aussparung (13) zwischen einem Fünftel und einem Sechstel der Höhe des kegelstumpfförmigen Bereichs (6) beträgt.

7. Gelenkpfanne (1) gemäß einem der vorhergehenden Ansprüche, wobei die Basis des Kanals (12) zumindest einen erhöhten Bereich (14) hat, der ein Mittel zum Widerstand gegen die Rotation bildet.

8. Gelenkpfanne (1) gemäß Anspruch 7, wobei die Basis des Kanals (12) um ihren Umfang eine im Wesentlichen epizykloidische Form aufweist, die zumindest eine Spitze (14) bildet.

9. Gelenkpfanne (1) gemäß Anspruch 8, wobei die Basis des Kanals (12) vier Spitzen (14) hat, die im Wesentlichem gleichförmig um den Umfang verteilt hat.

10. Gelenkpfanne (1) gemäß Anspruch 8, wobei die Basis des Kanals (12) zwei Spitzen (14) in im Wesentlichen gegenüberliegenden Positionen aufweist.

11. Gelenkpfanne (1) gemäß Anspruch 7, wobei die Basis des Kanals (12) drei Spitzen (14) aufweist, die voneinander jeweils im 120°-Winkel beabstandet sind.

12. Gelenkpfanne (1) gemäß einem der vorherigen Ansprüche, wobei der polare Bereich (2) der distalen Seite (4) abgeflacht ist.

13. Gelenkpfanne (1) gemäß einem der vorherigen Ansprüche, wobei die distale Seite (4) einen hohen Rauigkeitsgrad aufweist.

14. Gelenkpfanne (1) gemäß Anspruch 13, wobei die Rauigkeit durch ein Plasmasprühverfahren in Vakuum hergestellt wird.

15. Gelenkpfanne (1) gemäß Anspruch 13, wobei die Rauigkeit durch Korundstrahlen hergestellt wird.

16. Gelenkpfanne (1) gemäß Anspruch 1 mit drei in geeigneter Weise aufgeweiteten Löcher (11) zur Aufnahme von Schrauben (29) zur Verankerung der Gelenkpfanne (1) in dem Knochengewebe.

17. Gelenkpfanne (1) gemäß einem der vorhergehenden Ansprüche, aus Titan oder einer Titanlegierung hergestellt.

18. Hüftgelenkprothese umfassend eine Gelenkpfanne (1) gemäß Anspruch 1 und einen Gelenkeinsatz (16) aus einem synthetischen Material mit einer im Wesentlichen kappenförmigen Form, und einen Gelenksitz (20) zur Aufnahme eines kugelförmigen Kopfes (30) einer Oberschenkelprothese (31) umfassend, und eine Außenseite (18), die aus kegelstumpfförmigen Bereichen (21, 22, 23) besteht, die in Richtung eines polaren Bereichs (24) zulaufen, wobei der am proximalsten angeordnete kegelstumpfförmigen Bereich (21) einen im Wesentlichen ringförmigen Vorsprung(25) hat, der in einen Kanal (12) eingreifen kann, der in einem Sockel (1) vorgesehen ist, um den Gelenkeinsatz (16) mit der Gelenkpfanne (1) zu koppeln, **dadurch gekennzeichnet, dass** eine Nut (26) neben dem ringförmigen Vorsprung (25) in einer proximalen Richtung ausgebildet ist und sich um den gesamten Umfang des kegelstumpfförmigen Bereichs (21) erstreckt.

19. Hüftgelenkprothese gemäß Anspruch 18, wobei im Gelenkeinsatz (16) drei kegelstumpfförmige Bereiche (21, 22, 23) vorhanden sind.

20. Hüftgelenkprothese gemäß Anspruch 19, wobei in dem Gelenkeinsatz (16) der im Wesentlichen ringförmige Vorsprung (25) in etwa mittig relativ zur Höhe des am proximalsten angeordneten kegelstumpfförmigen Bereichs (21) angeordnet ist.

21. Hüftgelenkprothese gemäß einem der Ansprüche 18 bis 20, wobei im Gelenkeinsatz (16) der Vorsprung (25) in proximaler Richtung geneigt ist.

22. Hüftgelenkprothese gemäß Anspruch 18, wobei der Gelenkeinsatz (16) einen proximalen Flansch (17) umfasst.

23. Hüftgelenkprothese gemäß Anspruch 22, wobei im Gelenkeinsatz (16) der proximale Flansch (17) einen Bereich aufweist, der in proximaler Richtung verstärkt und vergrößert ist, damit der Gelenksitz (20) einem Auskugeln entgegenwirken kann.

24. Hüftgelenkprothese gemäß einem der Ansprüche 18 bis 23, wobei der Gelenkeinsatz (16) aus Polyäthylen hergestellt ist.

## Revendications

1. Emboîture (1) pour une prothèse articulaire de la hanche, l'emboîture (1) étant faite en une matière biocompatible et ostéo-intégrable, et possédant une forme comme une coiffe essentiellement sphérique, avec une surface distale (4) et une cavité proximale (5) appropriée pour accueillir à la fois des inserts articulaires (16) faits en une matière synthétique et des inserts articulaires (16) faits en une matière céramique, **caractérisée en ce que** la cavité (5) comprend des parties tronconiques (6, 7, 8), où la partie tronconique la plus proximale (6) a au moins un canal (12) essentiellement circonférentiel qui peut être accouplé à au moins une projection (25) respective se prolongeant depuis une surface extérieure (18) d'un insert articulaire (16) fait en une matière synthétique, **caractérisée en ce que** la partie tronconique la plus proximale (6) possède un évidement essentiellement cylindrique (13) adjacent au canal (12).

2. Emboîture (1) selon la revendication 1 dans laquelle l'évidement (13) est disposé sur le côté proximal du canal (12).

3. Emboîture (1) selon la revendication 2 dans laquelle le diamètre de l'évidement (13) correspond au diamètre de la partie tronconique (6) immédiatement adjacente.

4. Emboîture (1) selon la revendication 3 dans laquelle le canal (12) et l'évidement (13) sont disposés de manière approximativement centrale par rapport à la hauteur de la partie tronconique (6).

5. Emboîture (1) selon la revendication 4 dans laquelle la largeur globale du canal (12) et de l'évidement (13) est inférieure à un cinquième de la hauteur de la partie tronconique (6).

6. Emboîture (1) selon la revendication 5 dans laquelle la largeur globale du canal (12) et de l'évidement (13) se trouve entre un cinquième et un sixième de la hauteur de la partie tronconique (6).

7. Emboîture (1) selon l'une quelconque des revendications précédentes dans laquelle la base du canal (12) possède au moins une partie surélevée (14) constituant un moyen pour résister à la rotation.

8. Emboîture (1) selon la revendication 7 dans laquelle la base du canal (12) possède, autour de sa circonférence, une forme essentiellement épicycloïdale, formant au moins une cuspide (14).

9. Emboîture (1) selon la revendication 8 dans laquelle la base du canal (12) possède quatre cuspides (14) distribuées essentiellement uniformément autour de sa circonférence.

10. Emboîture (1) selon la revendication 8 dans laquelle la base du canal (12) possède deux cuspides (14) dans des positions essentiellement opposées.

11. Emboîture (1) selon la revendication 7 dans laquelle la base du canal (12) possède trois cuspides espacées par des angles de 120°.

12. Emboîture (1) selon l'une quelconque des revendications précédentes dans laquelle la région polaire (2) de la surface distale (4) est aplatie.

13. Emboîture (1) selon l'une quelconque des revendications précédentes dans laquelle la surface distale (4) a un degré de rugosité élevé.

14. Emboîture (1) selon la revendication 13 dans laquelle la rugosité est produite par la technique de pulvérisation par plasma sous vide.

15. Emboîture (1) selon la revendication 13 dans laquelle la rugosité est produite par décapage au corindon.

16. Emboîture (1) selon la revendication 1, possédant trois trous radiaux convenablement évasés (11) pour recevoir des vis (29) destinées à l'ancrage de l'emboîture (1) dans le tissu osseux.

17. Emboîture (1) selon l'une quelconque des revendications précédentes, faite en titane ou en alliage de titane.

18. Prothèse articulaire de la hanche comprenant une emboîture (1) selon la revendication 1 et un insert articulaire (16) fait en une matière synthétique ayant essentiellement une forme de coiffe et comprenant un siège articulaire (20) pour accueillir une tête sphérique (30) d'une prothèse fémorale (31), et une surface extérieure (18) composée de parties tronconiques (21, 22, 23) qui sont effilées vers une région polaire (24), dans laquelle la partie tronconique la plus proximale (21) possède une projection essentiellement annulaire (25) qui peut s'engager dans un canal (12) pourvu dans une emboîture (1) pour l'accouplement de l'insert articulaire (16) avec l'emboîture (1), **caractérisée en ce que**, une gorge (26) est formée en plus de la projection annulaire (25) dans une direction proximale, et s'étend autour de toute la circonférence de la partie tronconique (21).

19. Prothèse articulaire de la hanche selon la revendication 18 dans laquelle dans ledit insert articulaire (16) on trouve trois parties tronconiques (21, 22, 23).

20. Prothèse articulaire de la hanche selon la revendication 19 dans laquelle dans ledit insert articulaire (16) la projection essentiellement annulaire (25) est disposée de manière approximativement centrale par rapport à la hauteur de la partie tronconique la plus proximale (21).

21. Prothèse articulaire de la hanche selon l'une quelconque des revendications 18 à 20 dans laquelle dans ledit insert articulaire (16) la projection (25) est inclinée dans la direction proximale.

22. Prothèse articulaire de la hanche selon la revendication 18, dans laquelle ledit insert articulaire (16) comprend un volet proximal (16).

23. Prothèse articulaire de la hanche selon la revendication 22 dans laquelle dans ledit insert articulaire (16) le volet proximal (16) possède une région qui est renforcée et élargie dans la direction proximale, pour permettre au siège articulaire (20) de résister à la dislocation.

24. Prothèse articulaire de la hanche selon l'une quelconque des revendications 18 à 23, dans laquelle ledit insert articulaire (16) est fait en polyéthylène.
